# EUROPEAN PATENT APPLICATION

(11) **EP 2 387 983 A1**
(43) Date of publication of application: **23.11.2011**
(21) Application number: 10163435.0
(22) Date of filing: 20.05.2010
(51) Int. Cl.: A61G 7/018, G08B 21/02, H01H 35/02

(54) **Angle control alarm for a sickbed**

(71) Applicant: AOK Tooling Limited, Hong Kong (CN)
(72) Inventor: Teng, Mei Sheng, CHEUNG SHA WAN, KOWLOON (CN)
(74) Representative: Hellmich, Wolfgang

(57) **Abstract**

An angle control alarm for a sickbed comprises a housing and a alarm circuit, he alarm circuit comprises a control chip which has a first two pins electrically connected to a power source, a second two pins electrically connected to two alarm lights via two resistances, a third two pins electrically connected to a speaker by another two resistances, a seventh pin electrically connected to two gravity switches by a capacitance, and a eighth pin electrically connected to the power source by another capacitance. The angle control alarm can be used on different sickbeds, when the head of the sickbed is lowered to an angle less than 30 degrees, sound and light alarms will be activated to generate alarm signals.

## Description

### BACKGROUND OF THE INVENTION

### Field of the Invention

The present invention relates to an angle alarm, and more particularly to an angle control alarm for a sickbed.

### Description of the Prior Art

Some patients need to lie on an inclined bed, for example, a patient with pulmonary disease is required to raise his/her upper body about 30 degrees or more for easy breath. Conventionally, a sickbed is normally provided with a mechanical device which is capable of raising one end of the bed, however, raising the sickbed is all a matter of experience, therefore, the sickbed usually cannot be raised to a desired angle, which is bad for the patient.

The present invention has arisen to mitigate and/or obviate the afore-described disadvantages.

### SUMMARY OF THE INVENTION

The primary object of the present invention is to provide an angle control alarm for a sickbed, which is capable of monitoring the angle of the sickbed and giving out sound and light alarm signals.

To achieve the above object, an angle control alarm for a sickbed comprises a housing and a alarm circuit, he alarm circuit comprises a control chip which has a first two pins electrically connected to a power source, a second two pins electrically connected to two alarm lights via two resistances, a third two pins electrically connected to a speaker by another two resistances, a seventh pin electrically connected to two gravity switches by a capacitance, and a eighth pin electrically connected to the power source by another capacitance.

The gravity switches are ball or mercury switches.

The gravity switches are symmetrically arranged at an angle of 30 degrees with respect to a horizontal plane.

The gravity switches are symmetrically arranged at an angle of 30 degrees with respect to a horizontal plane.

The housing consists of an upper cover and a lower cover, inside the upper cover are provided the speaker, the gravity switches, a power switch, and a PCB, on the PCB are provided the control chip, the alarm lights, and a battery is disposed in a battery chamber defined in the lower cover and is electrically connected to the power switch and the PCB.

The upper cover is provided on its outer surface with a sound alarm window, a light alarm window, and an angle indicating icon.

Hooks and loops are glued to the lower cover.

The angle control alarm can be used on different sickbeds, when the head of the sickbed is lowered to an angle less than 30 degrees, sound and light alarms will be activated to generate alarm signals. It has been proven that raising the head of the sickbed to make the patient breathe better can reduce the risk of pneumonia. The angle control alarm of the present invention can improve the quality of taking care of the patients of such decease.

### BRIEF DESCRIPTION OF THE DRAWINGS

Fig. 1 is a perspective view of a part of an angle control alarm for a sickbed in accordance with the present invention;
Fig. 2 is an illustrative view of the installation of the power source of the angle control alarm for a sickbed in accordance with the present invention;
Fig. 3 shows the interior of the angle control alarm for a sickbed in accordance with the present invention;
Fig. 4 is an explode view of the angle control alarm for a sickbed in accordance with the present invention; and
Fig. 5 is a circuit diagram of the angle control alarm in accordance with the present invention.

### DETAILED DESCRIPTION OF THE PREFERRED EMBODIMENTS

The present invention will be clearer from the following description when viewed together with the accompanying drawings, which show, for purpose of illustrations only, the preferred embodiment in accordance with the present invention.

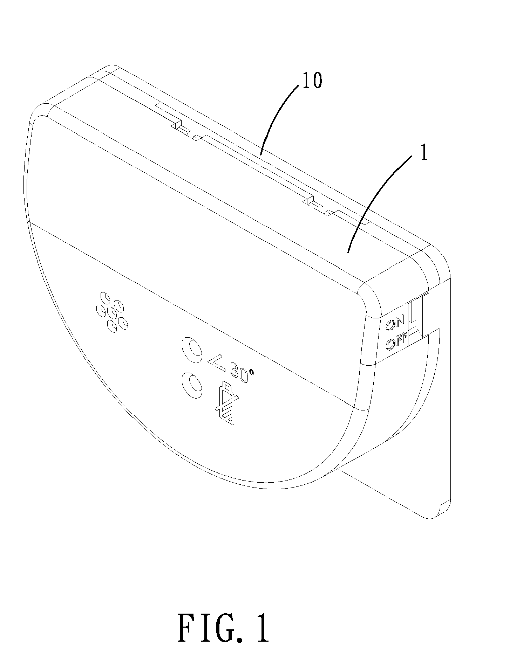

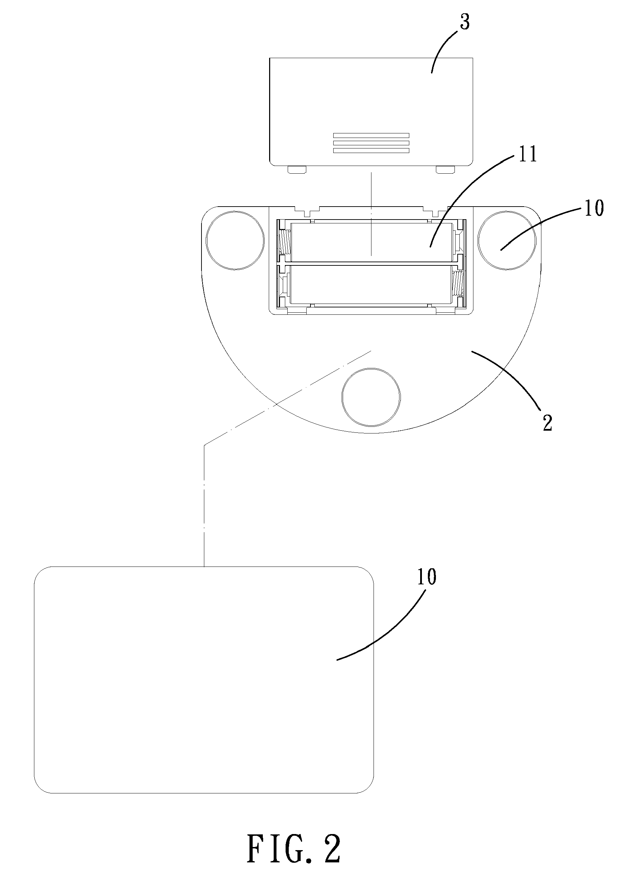

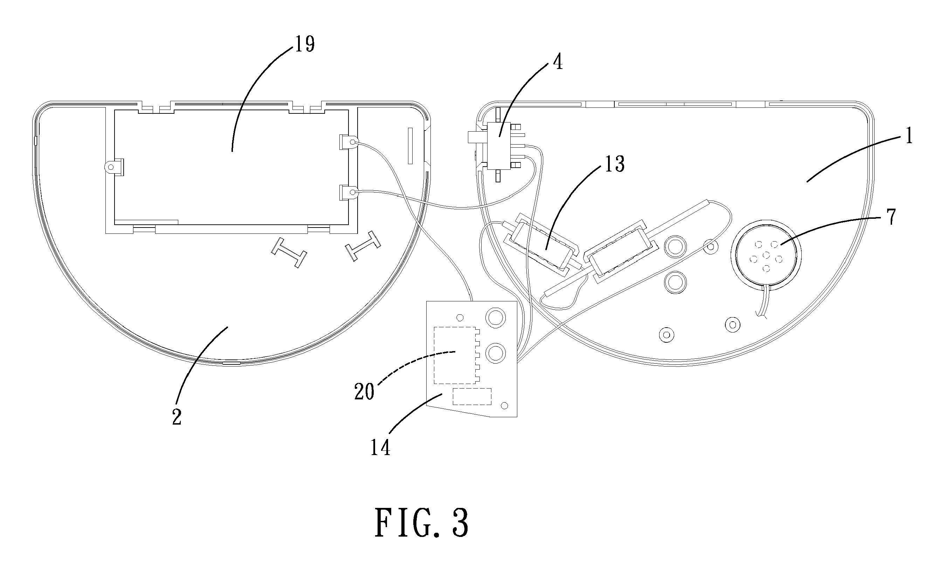

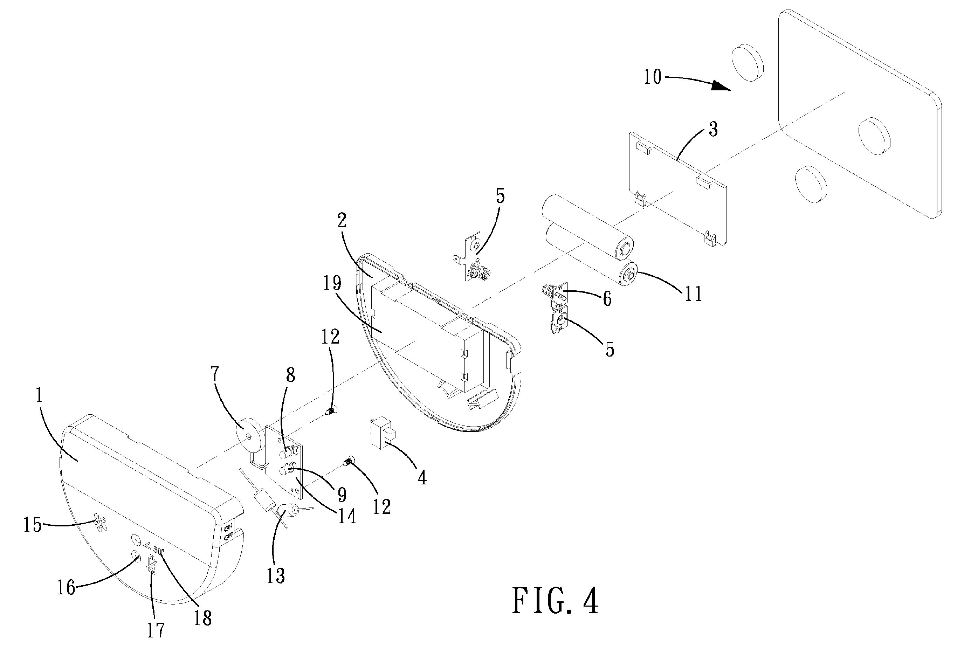

Referring to Figs. 1-4, an angle control alarm for a sickbed in accordance with the present invention comprises: an upper cover 1, a lower cover 2, a speaker 7, a red alarm light 8, a yellow alarm light 9, battery 11, ball switches 13, and printed circuit board (PCB) 14. The upper and lower covers 1, 2 are assembled together to form a housing. The upper cover 1 is provided on its outer surface with a sound alarm window 15, a light alarm window 16, a low voltage alarm icon 17, and an angle indicating icon 18. Inside the upper cover 1 are provided the speaker (SP) 7, the ball switches (S1, S2) 13, a power switch 4, and the PCB 14. On the PCB 14 are provided a control chip (u1) 20, the red alarm light (LED D2) 8, and the yellow alarm light (LED D1) 9. The battery 11 is disposed in a battery chamber 19 defined in the lower cover 2 and is electrically connected to the power switch 4 and the PCB 14.

This angle control alarm can be used on different sickbeds, when the head of the sickbed is lowered to an angle less than 30 degrees, sound and light alarms will be activated to generate alarm signals. It has been proven that raising the head of the sickbed to make the patient breathe better can reduce the risk of pneumonia. The angle control alarm of the present invention can improve the quality of taking care of the patients of such decease.

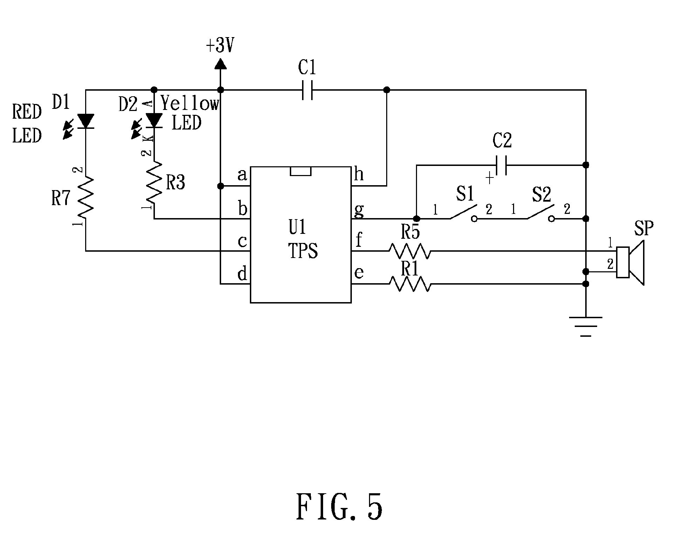

Fig. 5 is a circuit diagram of the angle control alarm in accordance with the present invention. The alarm circuit comprises a control chip has eight pin a, b, c, d, e, f, g and h. the two pins a and d are electrically connected to the power source, a second two b and c pins are electrically connected to two alarm lights LED D2 and LED D1 via two resistances R3 and R7, a third two pins e and f are electrically connected to the speaker SP by another two resistances R1 and R5, the seventh pin g is electrically connected to two gravity switches S1 and S2 by a capacitance C2, and the eighth pin h is electrically connected to the power source by another capacitance C1. The gravity switches S1 and S2 are ball switches or mercury switches.

When used in a sickbed, the upper surface of the angle control alarm in accordance with the present invention can be installed in parallel to any side of the head of the sickbed. When the head of the sickbed is lowered to an angle less than 30 degrees, the two ball switches S1, S2 are simultaneously switched on, the circuit is closed, and sound and light alarming signals will be produced. When the head of the sickbed is raised to an angle greater than 30 degrees, one of the two ball switches will be switched off, and the circuit is open, which means that the angle of the head of the sickbed is ok.

The principle of the circuit:

The ball switches S1 and S2 are symmetrically arranged at an angle of 30 degrees with respect to the horizontal plane and serially connected to each other and then electrically connected to a sound and light alarm circuit. When the two ball switches S1, S2 are simultaneously located at angle less than 30 degrees with respect to the horizontal plane, the two ball switches S1, S2 will all be switched on, and the whole circuit is closed and will generate alarm signals of blinking lights and sound. When any of the switches S1 and S2 is located at an angle equal to greater than 30 degrees with respect to the horizontal plane, the switch which is located at the angle equal to greater than 30 degrees will be switched off, and the whole circuit is open, and no alarm signals of sound and blinking lights will be generated.

If the accumulated alarm time exceeds 1 minute, the sound alarm will be stopped for 1 minute for the purpose of saving power. The life of the battery of the angle control alarm in accordance with the present invention is long up to at least 6 months.

When the battery voltage is lower than 2.3 V, the yellow alarm light will blink or flash at a frequency of 0.5 HZ to warn the user to replace the battery.

The angle control alarm in accordance with the present invention can be fixed to the head of the sickbed by hooks and loops or double sided tapes which are glued to the lower cover 2 for easy disassembly and maintenance.

While we have shown and described various embodiments in accordance with the present invention, it is clear to those skilled in the art that further embodiments may be made without departing from the scope of the present invention.

## Claims

1. An angle control alarm for a sickbed comprising a housing and a alarm circuit, **characterized in that**: the alarm circuit comprises a control chip which has a first two pins electrically connected to a power source, a second two pins electrically connected to two alarm lights via two resistances, a third two pins electrically connected to a speaker by another two resistances, a seventh pin electrically connected to two gravity switches by a capacitance, and a eighth pin electrically connected to the power source by another capacitance.

2. The angle control alarm for a sickbed as claimed in claim 1, wherein the gravity switches are ball or mercury switches.

3. The angle control alarm for a sickbed of one of claims 1 or 2, wherein the gravity switches are symmetrically arranged at an angle of 30 degrees with respect to a horizontal plane.

4. The angle control alarm for a sickbed of one of the preceding claims, wherein the housing consists of an upper cover and a lower cover, inside the upper cover are provided the speaker, the gravity switches, a power switch, and a PCB, on the PCB are provided the control chip, the alarm lights, and a battery is disposed in a battery chamber defined in the lower cover and is electrically connected to the power switch and the PCB.

5. The angle control alarm for a sickbed of claim 4, wherein the upper cover is provided on its outer surface with a sound alarm window, a light alarm window, and an angle indicating icon.

6. The angle control alarm for a sickbed of one of claims 4 or 5, wherein hooks and loops are glued to the lower cover.
